# EUROPEAN PATENT APPLICATION

(11) **EP 3 672 373 A1**
(43) Date of publication of application: **24.06.2020**
(21) Application number: 18215090.4
(22) Date of filing: 21.12.2018
(51) Int. Cl.: H05B 37/02, A61N 5/06, A61M 21/00

(54) **LIGHTING CONTROL**

(71) Applicant: Helvar Oy Ab, 02150 Espoo (FI)
(72) Inventor: HELLSTRÖM, Lars, 02150 ESPOO (FI); MAKRYGIANNIS, Sotirios, 00500 HELSINKI (FI)
(74) Representative: Berggren Oy, Helsinki & Oulu

(57) **Abstract**

According to an example embodiment, a lighting controller for controlling light output from one or more luminaires provided for illuminating a space is provided. The lighting controller is arranged to control the light output in accordance with a default lighting schedule that defines respective predefined durations for a sequence of alternating working periods and breaks, where the duration defined for a break in relation to the duration defined for the immediately preceding working period of the sequence increases over time, wherein the lighting controller is arranged to change at least one characteristic of the light output of the one or more luminaires upon the default lighting schedule indicating a transition from a working period to a break and upon the default lighting schedule indicating a transition from a break to a working period.

## Description

### TECHNICAL FIELD

The example and non-limiting embodiments of the present invention relate to lighting systems and, in particular, to controlling characteristics of the light output of a lighting system.

### BACKGROUND

Strict focus and concentration are essential for successfully carrying out tasks and assignments such as meetings, workshops, classroom training, studying, research work, etc.

Both everyday experience and formal studies suggest that an effective basic approach for ensuring sufficient focus and energy level involves repeatedly taking breaks to facilitate short-term recovery from the strain imposed by continuous cognitive effort. Hence, a known technique involves carrying out a task at hand according to a schedule that consists of alternating working periods and breaks.

While the basic framework for scheduling alternating working periods and breaks is readily applicable for assignments and tasks of different type, the most appropriate durations for the working periods and breaks depend on several factors, such as the type of the assignment/task, the number and characteristics of the individual(s) involved in carrying out the assignment/task, environmental conditions in the space in which the assignment/task is being carried out, the time of the day, etc. Moreover, an additional challenge arises from human factors associated with implementing enforcement of the scheduled alternating working periods and breaks.

### SUMMARY

It is an object of the present invention to provide a technique that facilitates enforcing a working schedule that involves alternating working periods and breaks in a manner that supports maintaining alertness and energy level of persons acting upon the working schedule.

According to an example embodiment, a lighting controller for controlling light output from one or more luminaires provided for illuminating a space is provided, the lighting controller arranged to control the light output in accordance with a default lighting schedule that defines respective predefined durations for a sequence of alternating working periods and breaks, where the duration defined for a break in relation to the duration defined for the immediately preceding working period of the sequence increases over time, wherein the lighting controller is arranged to change at least one characteristic of the light output of the one or more luminaires upon the default lighting schedule indicating a transition from a working period to a break and upon the default lighting schedule indicating a transition from a break to a working period.

According to another example embodiment, a method for controlling light output from one or more luminaires provided for illuminating a space is provided, the method comprising obtaining a default lighting schedule that defines respective predefined durations for a sequence of alternating working periods and breaks, where the duration defined for a break in relation to the duration defined for the immediately preceding working period of the sequence increases over time; and controlling the one or more luminaires to change at least one characteristic of their light output upon the default lighting schedule indicating a transition from a working period to a break and upon the default lighting schedule indicating a transition from a break to a working period.

According to another example embodiment, a computer program is provided, the computer program comprising computer readable program code configured to cause performing at least a method according to the example embodiment described in the foregoing when said program code is executed on a computing apparatus.

The computer program according to an example embodiment may be embodied on a volatile or a non-volatile computer-readable record medium, for example as a computer program product comprising at least one computer readable non-transitory medium having program code stored thereon, the program which when executed by an apparatus cause the apparatus at least to perform the operations described hereinbefore for the computer program according to an example embodiment of the invention.

The exemplifying embodiments of the invention presented in this patent application are not to be interpreted to pose limitations to the applicability of the appended claims. The verb "to comprise" and its derivatives are used in this patent application as an open limitation that does not exclude the existence of also unrecited features. The features described hereinafter are mutually freely combinable unless explicitly stated otherwise.

Some features of the invention are set forth in the appended claims. Aspects of the invention, however, both as to its construction and its method of operation, together with additional objects and advantages thereof, will be best understood from the following description of some example embodiments when read in connection with the accompanying drawings.

### BRIEF DESCRIPTION OF FIGURES

The embodiments of the invention are illustrated by way of example, and not by way of limitation, in the figures of the accompanying drawings, where
Figure 1 illustrates a lighting system according to an example;
Figure 2 illustrates a method according to an example; and
Figure 3 illustrates a block diagram of some elements of an apparatus according to an example.

### DESCRIPTION OF SOME EMBODIMENTS

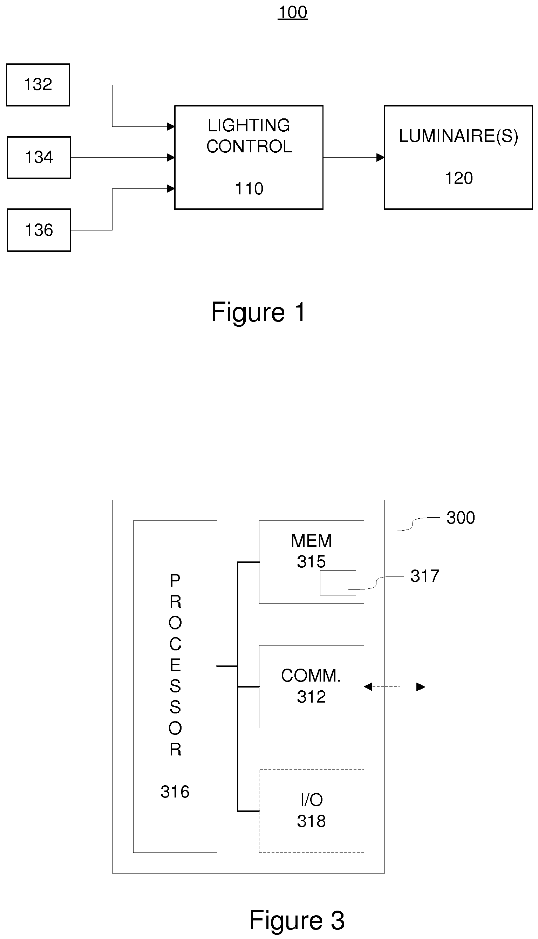

Figure 1 illustrates a block diagram of some components of a lighting system 100 according to an example. The lighting system 100 depicted in Figure 1 comprises one or more luminaires 120 for illuminating a space, one or more environmental sensors 132, one or more presence sensors 134 and one or more cameras 136 for providing respective sensor signals that are descriptive of respective characteristics in the space, and a lighting controller 110 for controlling light output from the one or more luminaires 120.

Although depicted in the example of Figure 1, the lighting system 100 may be provided without one or more of the environmental sensor(s) 132, the presence sensor(s) 134 and the camera(s) 136. If present, the one or more environmental sensors 132 may comprise, for example, one or more CO₂ sensors, one or more humidity sensors, one or more temperature sensors, etc. If present, the one or more presence sensors 134 may comprise, for example, respective passive infrared (PIR) sensors. If present, the one or more cameras 136 may comprise, for example, respective one or more digital video cameras or thermal cameras that are able provide respective (digital) images at a predefined (high or low) image resolution. If the respective sensor(s) are present, the lighting controller 110 may be arranged to control the light output from the one or more luminaires 160 based at least in part on sensor signals obtained from the environmental sensor(s) 132, the presence sensor(s) 134 and/or the camera(s) 136, as described in the following.

The lighting controller 110 is arranged to control the light output from the one or more luminaires 120 in accordance with a lighting schedule that defines a sequence of alternating working periods and breaks such that the relative duration of the break in comparison to the duration of the immediately preceding working period increases over time. In other words, the relative duration of the working period in comparison to the duration of the immediately following break decreases over time. Such a change in the relative duration over time may be provided via defining working periods of the sequence to have absolute durations that get shorter over time and/or via defining breaks of the sequence to have absolute durations that get longer over time. In an example, in the sequence defined by the lighting schedule the predefined duration of the working periods gradually decreases over time, whereas the predefined duration of the breaks is kept constant over time. In another example, the predefined duration of the working periods is gradually decreased over time, whereas the predefined duration of the breaks is gradually increased over time. In further example, the predefined duration of the working periods is kept constant over time while the predefined duration of the breaks is gradually increased over time.

The absolute predefined durations of the working periods and the breaks of the lighting schedule may be varied within a respective predefined range. In an example, the duration of a working period may be within a range from 5 minutes to 30 minutes, whereas the duration of a break may be within a range from 5 to 15 minutes. In an example, the initial working period duration may be in a range from 20 to 30 minutes, e.g. 25 minutes, and/or the initial break duration may be in a range from 3 to 10 minutes, e.g. 5 minutes. In an example, the duration of a break does not exceed the duration the immediately preceding working period of the sequence. As a further example, a lighting schedule may define a sequence that involves a working period of 25 min followed by a break of 5 min for the first two hours of the lighting schedule, a working period of 15 min followed by a break of 5 min for the following two hours of the lighting schedule, and a working period of 10 min followed by a break of 5 min for the subsequent hours of the lighting schedule. Hence, in this example the duration of the breaks stays constant over time, where the duration of the working periods is gradually decreased over time.

The timing for a sequence of alternating working periods and breaks according to a lighting schedule may be defined in one of a plurality of ways, non-limiting examples in this regard including the following:
- A lighting schedule defines respective absolute durations for a plurality of working periods and breaks included therein;
- A lighting schedule defines respective reference durations for the first working period and for the first break and further defines, for subsequent working periods and breaks, a respective difference to the respective reference duration;
- A lighting schedule defines one or more rules for defining respective absolute durations for a plurality of working periods and breaks included therein as a function of time and/or as a function of their position in the sequence of alternating working periods and break.

The lighting controller 110 is arranged to apply the lighting schedule by operating the one or more luminaires 120 to change at least one characteristic of the light output upon the lighting schedule indicating a transition from a working period to a break and upon the lighting schedule indicating a change from a break to a working period. Non-limiting examples of a change in the light output characteristics upon a transition from a working period to a break or vice versa include a change of light intensity level, a change in color temperature of the light output and a change in color of the light output.

In an example, the change of the light output characteristics is applied over the duration of the breaks, e.g. such that the lighting controller 110 applies the lighting schedule by controlling the one or more luminaires 120 to provide the light output according to first light output characteristics throughout a working period and by controlling the one or more luminaires 120 to provide the light output according to second light output characteristics throughout a break. In another example, the change of the light output characteristics is applied in the beginning of each working period and break (possibly with the exception of the first working period), e.g. such that the lighting controller 110 applies the lighting schedule by controlling the one or more luminaires 120 to provide the light output according to first light output characteristics during a first predefined sub-period in the beginning of a working period and to provide the light output according to second light output characteristics for the remaining part of the working period, and by controlling the one or more luminaires 120 to provide the light output according to the first light output characteristics during a second predefined sub-period in the beginning of a break and to provide the light output according to the second light output characteristics for the remaining part of the working period.

Further non-limiting example scenarios for changing the light output characteristics between working periods and breaks of a lighting schedule are provided in the following.
- The light intensity level applied during a working period may be a predefined nominal light intensity level (e.g. 60 % of the maximum light intensity level of the one or more luminaires 120) or a predefined low light intensity level (e.g. 10 to 20 % of the maximum light intensity level of the one or more luminaires 110 to provide a 'presentation mode' in a meeting room, a classroom, an auditorium, etc.), whereas the light intensity level applied during a break may be the maximum light intensity level of the one or more luminaires 120. In a variation of this example, the maximum light intensity may be applied for a respective predefined sub-period of time in the beginnings of the working periods and breaks, whereas the predefined nominal or low light intensity level may be applied for the remaining parts of the working periods and breaks.
- The color temperature of the light output applied during a working period may be a low color temperature e.g. in a range from 2700 to 3000 K, whereas the color temperature applied during a break may be a high color temperature over 5000 K, e.g. 6500 K. In a variation of this example, the high color temperature may be applied for a respective predefined sub-period of time in the beginnings of the working periods and breaks, whereas the low color temperature may be applied for the remaining parts of the working periods and breaks.
- The color of the light output applied during a working period may be white (or, in general, a first color), whereas the color of light output applied during a break may be non-white (or, in general, a second color that is different from the first color). In a variation of this example, the white light (or the first color) may be applied for a respective predefined sub-period of time in the beginnings of the working periods and breaks, whereas the non-white light (or the second color) may be applied for the remaining parts of the working periods and breaks.

The lighting controller 110 applying a certain lighting schedule once is referred to herein as a lighting session. Aspects related to the lighting controller 110 selecting a lighting schedule for a lighting session and initiating and terminating the lighting session are described in the following via non-limiting examples.

In an example, the lighting controller 110 may be provided with a (single) predefined lighting schedule defined for the lighting controller 110 and hence a given lighting session may (always) be based on this predefined lighting schedule. It should be noted also, however, that even the text herein refers to a predefined lighting schedule, the predefined lighting schedule serves as a default lighting schedule that provides the baseline for the lighting controller 110 implementing a given lighting session, whereas respective durations of one or more working periods and/or breaks of the default lighting schedule may be adjusted in the course of the given lighting session, as described in the following via non-limiting examples.

In another example, the lighting controller 110 provides a plurality (i.e. two or more) of different predefined lighting schedules and one of the plurality of the lighting schedules is selected as the default lighting schedule that serves as basis for the given lighting session via a user interface coupled to the lighting controller 110. In a further example, a user of the lighting system 100 may define characteristics of a default lighting schedule to be applied as basis for the given lighting session via the user interface coupled to the lighting controller 110. Herein, the user interface may be provided, for example, as a control panel that includes one or more buttons and/or a touch-sensitive panel or display, via which a user may enter commands that result in selection or definition of the default lighting schedule. According to an example, the control panel may comprise a physical control panel that may be installed e.g. in a wall or other fixed structure in the space in which lighting system 100 is operated, which physical control panel is communicatively coupled to the lighting controller 110 via a wired or wireless communication link. In another example, a physical control panel may be provided in a dedicated remote control device that may be communicatively coupled to the lighting controller 110 via a wireless communication link. According to another example, the control panel may comprise a virtual control panel provided via a graphical user interface of a computing device such as a mobile phone, a tablet computer, in a laptop computer, etc. that is communicatively coupled to the lighting controller 110 via a wireless communication link and that is arranged to run a software application configured to provide the virtual control panel and to transfer the user commands received therethrough to the lighting controller 110 via the wireless communication link.

The lighting session based on the default lighting schedule may be initiated either manually or automatically. Manual initiation may involve the user entering an activation command via the user interface communicatively coupled to the lighting controller 120. As an example in this regard, the user interface may provide the user with a dedicated user interface element that enables initiating (or terminating) the applied lighting schedule. In other example, the user selecting or defining the applied default lighting schedule may implicitly serve as an activation command that results in initiation of the default lighting schedule.

As an example, automatic initiation may involve the lighting controller 110 initiating the lighting session based at least in part on information received from the one or more presence sensors 134 and/or the one or more cameras 136 in combination with external scheduling information. As an example, the external scheduling information may be based on a calendar entry pertaining to the space (e.g. a meeting room, a classroom, an auditorium, etc.) in which the lighting system 100 is employed, which calendar entry may provide an indication of the starting time and ending time of a session booked in the space and possibly also an indication of the number of persons involved in the booking. Consequently, the automatic initiation of the respective lighting session may comprise the lighting controller 110 receiving an indication of the starting time for a booking and initiating the lighting session in response to respective sensor signal(s) obtained from the presence sensor(s) 134 and/or from the camera(s) 136 indicating presence of one or more persons in the space at the indicated starting time. Herein, the external scheduling information may originate from a calendar implemented in or otherwise available for the lighting controller 110 (and accessible e.g. via the user interface coupled to the lighting controller 110) or from calendar application provided in a computing device (such a mobile phone, a tablet computer, a laptop computer, etc.) communicatively coupled to the lighting controller 110.

In an example, once initiated, the lighting controller 110 may be arranged to control provision of the light output from the one or more luminaires 120 in accordance with the applied default lighting schedule for a predefined period of time. In this regard, the lighting schedule may further define a predefined overall duration or a direct or indirect indication of the predefined overall duration for the lighting schedule may be received as part of the external scheduling information, e.g. as the ending time indicated for the calendar entry of pertaining to the space in which the lighting system 100 is employed. In another example, the lighting controller 110 may be arranged to continue provision of the light output in accordance with the applied default lighting schedule until instructed otherwise, e.g. until receiving a termination command. The termination command may involve the user entering the termination command via the user interface communicatively coupled to the lighting controller 120, e.g. via operating a dedicated user interface element that enables terminating (or initiating) the currently applied lighting schedule. In a further example, the lighting controller 110 may be arranged to terminate provision of the light output in response to the respective sensor signals from the one or more presence sensors 134 and/or from the one or more cameras 136 indicating that no persons are present in the space or in response to the respective sensor signals having indicated absence of persons in the room for at least a predefined period of time.

The lighting controller 110 may be further arranged to adjust timing and/or respective predefined durations defined for one or more working periods and/or for one or more breaks of the default lighting schedule applied for the current lighting session based at least in part on respective sensor signals received from the one or more environmental sensors 132. The adjustment may take place in the course of the (current) lighting session and it may comprise one of increasing or decreasing a duration defined in the default lighting schedule for a working period or for a break in relation to the duration defined for another working period or break of the default schedule and/or increasing or decreasing a duration defined in the default schedule for a working period or a break in relation to the overall duration of the lighting session in which the default lighting schedule is applied. As an example, the adjustment may comprise one of increasing or decreasing the duration defined in the default lighting schedule for a given working period in relation to the duration of the break that immediately precedes the given working period, in relation to the duration of the break that immediately follows the given working period, or in relation to the overall duration of the lighting session. In the following such adjustment is referred as an adjustment of relative duration of a working period.

Non-limiting examples of using the information obtained in respective sensors signals received from the one or more environmental sensors 132 for decreasing respective relative durations of one or more working periods defined in the default lighting schedule are provided in the following:
- The lighting controller 110 may be arranged to decrease the relative duration of at least one working period in response to the sensor signal(s) from the CO₂ sensor(s) indicating a CO₂ level that exceeds a reference CO₂ level. Herein, the reference CO₂ level may comprise, for example, a predefined absolute reference CO₂ level or a relative reference CO₂ level in comparison to a CO₂ level measured in the beginning of the lighting session or in the beginning of the current working period.
- The lighting controller 110 may be arranged to decrease the relative duration of at least one working period in response to the sensor signal(s) from the humidity sensor(s) indicating a humidity level that exceeds a reference humidity level. Herein, the reference humidity level may comprise, for example, a predefined absolute reference humidity level or a relative reference humidity level in comparison to a humidity level measured in the beginning of the lighting session or in the beginning of the current working period.
- The lighting controller 110 may be arranged to decrease the relative duration of at least one working period in response to the sensor signal(s) from the temperature sensor(s) indicating a temperature that exceeds a reference temperature. Herein, the reference temperature may comprise, for example, a predefined absolute reference temperature or a relative reference temperature in comparison to the temperature measured in the beginning of the lighting session or in the beginning of the current working period.

In the examples above, decreasing the relative duration of at least one working period may comprise decreasing the relative duration of a single working period or decreasing respective relative durations of a plurality of working periods. In this regard, the lighting controller 110 decreasing the relative duration of at least one working period may comprise decreasing the relative duration of the working period during which the respective one of the CO₂ level, humidity or temperature was found to exceed the respective threshold level and/or decreasing the relative duration of one or more subsequent working periods . Consequently, an effect arising from an environmental condition that can be excepted to result in more rapid decrease of focus and energy level under a continuous cognitive effort is compensated by decreasing respective relative durations of one or more working periods to shorten the time period(s) that require continuous cognitive effort and/or to allow more time for short-term recovery from the strain imposed by the continuous cognitive effort.

Along the lines described above, the adjustment of the default lighting schedule by decreasing the relative duration of a given working period may involve decreasing the duration defined for the given working period in relation to the duration defined for the immediately preceding and/or immediately following break and/or in relation to the overall duration of the lighting session in which the default lighting schedule is applied. Non-limiting examples for shortening the relative duration defined for a given working period include the following:
- shortening the absolute duration defined for the given working period in the default lighting schedule while keeping the duration defined for the break that immediately precedes and/or for the break that immediately follows the given working period unchanged,
- keeping the absolute duration defined for the given working period in the default lighting schedule unchanged while increasing the absolute duration defined for the break that immediately precedes the given working period and/or for the break that immediately follows the given working period,
- shortening the absolute duration defined for the given working period in the default lighting schedule and increasing the absolute duration defined for the break that immediately precedes the given working period and/or for the break that immediately follows the given working period,
- shortening the absolute duration defined for the given working period in the default lighting schedule and shortening the absolute duration defined for one or both of the break that immediately precedes the given working period and/or the break that immediately follows the given working period.

In the examples above, shortening the absolute duration defined for the given working period, increasing or shortening the absolute duration defined for the immediately preceding and/or for the immediately following break may involve shortening or increasing (as applicable) by a respective predefined absolute amount (defined e.g. by minutes and/or seconds) or by shortening or increasing (as applicable) by a respective relative amount (defined e.g. as a percentage of the respective duration defined by the default lighting schedule).

In the examples above concerning adjustment of period durations defined in the default lighting schedule by decreasing the relative duration of at least one working period, the lighting controller 110 may be further arranged to issue and/or transmit a command for a HVAC system or an air conditioning system applied in the space to operate at a higher speed during one or more breaks that follow the working period during which the respective one of the CO₂ level, humidity or temperature was found to exceed the respective threshold level.

The examples provided in the foregoing describe decreasing the relative duration of one or more working periods based at least in part on information received in respective sensors signals received from the one or more environmental sensors 132. Further examples of using the information obtained from the one or more environmental sensors 132 include increasing the relative duration of one or more working periods based at least in part on information received in respective sensors signals received from the one or more environmental sensors 132. In this regard, the adjustment of relative duration of a working period may be triggered, for example in response to a sensor signal from a CO₂ sensor indicating decrease in CO₂ level, in response to a sensor signal from a humidity sensor indicating decrease in humidity level, and/or in response to a sensor signal from a temperature sensor indicate decrease in temperature.

A more detailed example in this regard pertaining to adjustment of the relative duration of at least one working period in dependence of information received in the sensor signal(s) from the CO₂ sensors is provided in the following. This example readily generalizes into increasing the relative duration of at least one working period in in dependence of information received in the respective sensor signals from environmental sensors 132 of other type (e.g. the one or more humidity sensors and/or the one or more temperature sensors), *mutatis mutandis.* In this regard, the lighting controller 110 may be arranged to increase the relative duration of at least one working period in response to the sensor signal(s) from the CO₂ sensor(s) indicating an decrease in CO₂ level that exceeds a predefined threshold level. The decreased CO₂ level may be evaluated, for example in comparison to the CO₂ level measured in the beginning of the lighting session or in the beginning of the current working period. Along the lines described for decreasing the relative duration of a given working period, increasing the relative duration of the working period may comprise increasing of the relative duration of the working period during which the decreased CO₂ level was detected and/or that of one or more subsequent working periods, whereas increasing the relative duration of a given working period may comprise, for example, increasing the absolute duration defined for the given working period and/or decreasing the absolute duration defined for one or both of the break that immediately precedes the given working period and the break that immediately follows the given working period.

The lighting controller 110 may be further arranged to adjust timing and/or respective durations defined for one or more working periods and/or for one or more breaks of the default lighting schedule applied for the current lighting session based at least in part on respective sensor signals received from the one or more cameras 136 that arranged to capture images of the space in which the lighting system 100 is applied.

As an example in this regard, lighting controller 110 may apply (or employ another device to apply) image analysis on one or more images obtained from the camera(s) 136 to estimate the number of people depicted in the one or more images and adjust the duration defined in the default lighting schedule for one or more working periods in dependence of the estimated number of people. As an example, this may involve decreasing the relative duration of the current working period and/or the relative duration of one or more subsequent working periods in response to image analysis indicating that the number of persons in the space exceeds a predefined threshold and/or increasing the relative duration of the current working period and/or that of one or more subsequent working periods in response to image analysis indicating that the number of persons in the space is below the predefined threshold.

As another example pertaining to the adjustment of respective durations defined for one or more working periods and/or one or more breaks of the default lighting schedule based at least in part on respective sensor signals received from the one or more cameras 136 involves the lighting controller 110 applying (or employing another device to apply) image analysis on one or more images obtained from the camera(s) 136 to estimate the mood of the person depicted in the image(s). The lighting controller 110 may be arranged to decrease relative duration defined for the current working period and/or for one or more subsequent working periods in response to such mood analysis suggesting low energy. In contrast, the lighting controller 110 may be arranged to keep duration defined for the current and/or for subsequent working periods unchanged or to increase the relative duration defined for the current working period and/or for one or more subsequent working periods in response to the mood analysis suggesting high energy.

The lighting controller 110 may be further arranged to adjust timing and/or respective durations of one or more working periods and/or one or more breaks of the lighting schedule applied for the current lighting session based at least in part on respective indications received from external user-specific devices, such as a mood ring or an activity tracker, or a mobile phone application operating to indicate the mood of the user. The lighting controller 110 may be arranged to decrease the relative duration defined for the current working period and/or for one or more subsequent working periods in response to one or more indications received from user-specific devices (e.g. ones worn by a person in the space) suggesting low energy or low activity. In contrast, the lighting controller 110 may be arranged to keep the duration defined for the current working period and/or for subsequent working periods unchanged or to increase the relative duration defined for the current working period and/or for one or more subsequent working periods in response to one or more indications received from user-specific devices suggesting high energy or high activity.

The lighting controller 110 may be arranged to collect the sensor data from the environmental sensor(s) 132, the presence sensor(s) 134 and the camera(s) 136 to extent they are present in the lighting system 100 over a plurality of lighting sessions and store the collected sensor data e.g. in a memory or a mass storage device accessible by the lighting controller 110. The sensor data may be further accompanied by timing adjustment data comprising information concerning adjustments made to relative durations defined for working periods and/or breaks defined in the applicable default lighting schedule based on the respective sensor data. The sensor data, possibly together with the timing adjustment data, may be referred to as history data, which may be provided separately for each predefined lighting schedule available in the lighting controller 110. Hence, the history data for a given predefined lighting schedule may include, for example, respective indications of observed CO₂ level, humidity level and/or temperature as a function of time for a plurality of past lighting sessions. The history data may further comprise respective indications of the number of persons in the space for the plurality of the past lighting sessions.

Once a sufficient amount of history data is available, the lighting controller 110 may be arranged to analyze the history data to estimate the change in value of an environmental parameter (e.g. the CO₂ level, the humidity level, the temperature) over time (since beginning of the lighting session) in view of the number of persons in the space and the resulting adjustment to be applied for durations of the working periods and/or breaks defined in the respective predefined lighting schedule. Consequently, according to an example, upon initiating a lighting session that as based on a certain predefined lighting schedule, the lighting controller 110 may apply this information to adapt durations defined in the certain predefined lighting schedule for its working periods and breaks accordingly, thereby creating a respective modified default lighting schedule that may be applied for the lighting session instead of the original one. With such an approach, there is no need for adjusting the durations defined for the respective original predefined lighting schedule in the course of the lighting session and hence it is possible to provide a lighting schedule that takes into account the expected changes in environmental conditions even without monitoring or having access to the respective sensor signals from the environmental sensor(s) 132.

The lighting system 100 and its operation under control of the lighting controller 110 described in foregoing is applicable in spaces that are intended for tasks or assignments that are typically require continued focus and concentration. Non limiting examples of such spaces include meeting rooms, office rooms, classrooms, auditoriums, etc. On the other hand, the lighting system 100 and the associated lighting control (by operation of the lighting controller 110) are equally applicable in spaces of more private nature, such as hotel rooms and private homes as well.

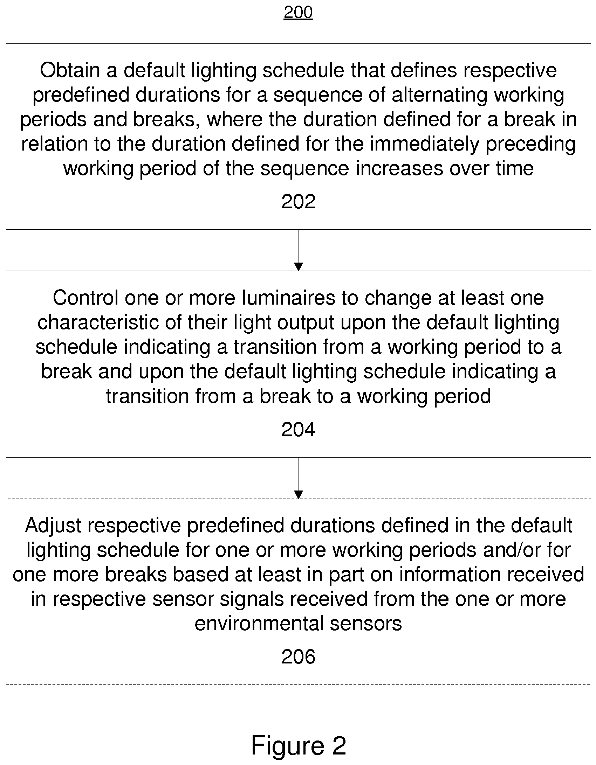

In the foregoing, aspects related to provision of light output from the one or more luminaires 120 in accordance with the lighting schedule that defines alternating working periods and breaks has been described with references to the lighting system 100 and to the lighting controller 110. The respective operation may be, alternatively, described as steps of method 200 illustrated by the flowchart depicted in Figure 2, where the method 200 is provided for controlling light output from the one or more luminaires 120 arranged for illuminating a space.

The method 200 comprises obtaining a default lighting schedule that defines respective predefined durations for a sequence of alternating working periods and breaks, where the duration defined for a break in relation to the duration defined for the immediately preceding working period of the sequence increases over time, as indicated in block 202, and controlling the one or more luminaires 120 to change at least one characteristic of their light output upon the default lighting schedule indicating a transition from a working period to a break and upon the default lighting schedule indicating a transition from a break to a working period, as indicated in block 204. The method 200 may further comprise, for example, adjusting respective predefined durations defined in the default lighting schedule for one or more working periods and/or for one more breaks based at least in part on information received in respective sensor signals received from the one or more environmental sensors 132, as indicated in block 206.

The method 200 may be (further) varied in a number of ways, for example in view of the examples concerning operation of the lighting controller 110 described in the foregoing.

Figure 3 illustrates a block diagram of some components of an exemplifying apparatus 300. The apparatus 300 may comprise further components, elements or portions that are not depicted in Figure 3. The apparatus 300 may be employed e.g. in implementing at least some aspects of the lighting controller 110 described in the foregoing. The apparatus 300 comprises a processor 316 and a memory 315 for storing data and computer program code 317. The memory 315 and a portion of the computer program code 317 stored therein may be further arranged to, with the processor 316, to implement at least some of the operations, procedures and/or functions described in the foregoing in context of the lighting controller 110 described in the foregoing.

The apparatus 300 comprises a communication portion 312 for communication with other devices. The communication portion 312 comprises at least one communication apparatus that enables wired or wireless communication with other apparatuses. A communication apparatus of the communication portion 312 may also be referred to as a respective communication means.

The apparatus 300 may further comprise user I/O (input/output) components 318 that may be arranged, possibly together with the processor 316 and a portion of the computer program code 317, to provide a user interface for receiving input from a user of the apparatus 300 and/or providing output to the user of the apparatus 300 to control at least some aspects of operation of the lighting controller 110 implemented by the apparatus 300. The user I/O components 318 may comprise hardware components such as a display, a touchscreen, a touchpad, a mouse, a keyboard, and/or an arrangement of one or more keys or buttons, etc. The user I/O components 318 may be also referred to as peripherals. The processor 316 may be arranged to control operation of the apparatus 300 e.g. in accordance with a portion of the computer program code 317 and possibly further in accordance with the user input received via the user I/O components 318 and/or in accordance with information received via the communication portion 312.

Although the processor 316 is depicted as a single component, it may be implemented as one or more separate processing components. Similarly, although the memory 315 is depicted as a single component, it may be implemented as one or more separate components, some or all of which may be integrated/removable and/or may provide permanent / semi-permanent/ dynamic/cached storage.

The computer program code 317 stored in the memory 315, may comprise computer-executable instructions that control one or more aspects of operation of the apparatus 300 when loaded into the processor 316. As an example, the computer-executable instructions may be provided as one or more sequences of one or more instructions. The processor 316 is able to load and execute the computer program code 317 by reading the one or more sequences of one or more instructions included therein from the memory 315. The one or more sequences of one or more instructions may be configured to, when executed by the processor 316, cause the apparatus 300 to carry out at least some of the operations, procedures and/or functions described in the foregoing in context of the lighting controller 110.

Hence, the apparatus 300 may comprise at least one processor 316 and at least one memory 315 including the computer program code 317 for one or more programs, the at least one memory 315 and the computer program code 317 configured to, with the at least one processor 316, cause the apparatus 300 to perform at least some of the operations, procedures and/or functions described in the foregoing in context of the lighting controller 110.

The computer program(s) stored in the memory 315 may be provided e.g. as a respective computer program product comprising at least one computer-readable non-transitory medium having the computer program code 317 stored thereon, the computer program code, when executed by the apparatus 300, causes the apparatus 300 at least to perform at least some of the operations, procedures and/or functions described in the foregoing in context of the lighting controller 110. The computer-readable non-transitory medium may comprise a memory device or a record medium such as a CD-ROM, a DVD, a Blu-ray disc or another article of manufacture that tangibly embodies the computer program. As another example, the computer program may be provided as a signal configured to reliably transfer the computer program.

Reference(s) to a processor should not be understood to encompass only programmable processors, but also dedicated circuits such as field-programmable gate arrays (FPGA), application specific circuits (ASIC), signal processors, etc. Features described in the preceding description may be used in combinations other than the combinations explicitly described.

In the present disclosure, although functions have been described with reference to certain features, those functions may be performable by other features whether described or not. Although features have been described with reference to certain embodiments, those features may also be present in other embodiments whether described or not.

## Claims

1. A lighting controller (110) for controlling light output from one or more luminaires (120) provided for illuminating a space, the lighting controller (110) arranged to:
control the light output in accordance with a default lighting schedule that defines respective predefined durations for a sequence of alternating working periods and breaks, where the duration defined for a break in relation to the duration defined for the immediately preceding working period of the sequence increases over time,
wherein the lighting controller (110) is arranged to change at least one characteristic of the light output of the one or more luminaires (120) upon the default lighting schedule indicating a transition from a working period to a break and upon the default lighting schedule indicating a transition from a break to a working period.

2. A lighting controller (110) according to claim 1, wherein in the default lighting schedule the increase in the relative duration defined for a break in comparison to the duration defined for the immediately preceding working period of the sequence is provided by one or more of the following:
the duration defined for a working period decreases over time,
the duration defined for a break increases over time.

3. A lighting controller (110) according to claim 1 or 2, further arranged to:
control the one or more luminaires (120) to provide light output according to first light output characteristics throughout a working period; and
control the one or more luminaires (120) to provide light output according to second light output characteristics throughout a working period.

4. A lighting controller (110) according to claim 1 or 2, further arranged to:
control the one or more luminaires (120) to provide light output according to first light output characteristics during a first predefined sub-period in the beginning of a working period and to provide light output according to second light output characteristics for the remaining part of the working period; and
control the one or more luminaires (120) to provide light output according to the first light output characteristics during a second predefined sub-period in the beginning of a break and to provide light output according to the second light output characteristics for the remaining part of the break.

5. A lighting controller (110) according to any of claims 1 to 4, wherein the change of at least one characteristic of the light output upon a transition between a working period and a break comprises at least one of the following:
a change in light intensity level,
a change in color temperature of the light output,
a change in color of the light output.

6. A lighting controller (110) according to any of claims 1 to 5, further arranged to adjust respective predefined durations defined in the default lighting schedule for one or more working periods and/or for one more breaks based at least in part on information received in respective sensor signals received from one or more environmental sensors (132).

7. A lighting controller (110) according to claim 6, arranged to decrease the relative duration of at least one working period in response to a sensor signal received from a CO₂ sensor indicates CO₂ level that exceeds a reference CO₂ level.

8. A lighting controller (110) according to claim 6 or 7, arranged to decrease the relative duration of at least one working period in response to a sensor signal received from a humidity sensor indicates humidity level that exceeds a reference humidity level.

9. A lighting controller (110) according to any of claims 6 to 8, arranged to decrease the relative duration of at least one working period in response to a sensor signal received from a temperature sensor indicates temperature that exceeds a reference temperature.

10. A lighting controller (110) according to any of claims 6 to 9, wherein decreasing the relative duration of a given working period comprises one of the following:
shortening the absolute duration defined for the given working period in the default lighting schedule while keeping the duration defined for the break that immediately precedes and/or for the break that immediately follows the given working period unchanged,
keeping the absolute duration defined for the given working period in the default lighting schedule unchanged while increasing the absolute duration defined for the break that immediately precedes the given working period and/or for the break that immediately follows the given working period,
shortening the absolute duration defined for the given working period in the default lighting schedule and increasing the absolute duration defined for the break that immediately precedes the given working period and/or for the break that immediately follows the given working period,
shortening the absolute duration defined for the given working period in the default lighting schedule and shortening the absolute duration defined for the break that immediately precedes the given working period and/or for the break that immediately follows the given working period.

11. A lighting controller (110) according to any of claims 6 to 10, arranged to increase the relative duration of at least one working period in response to a sensor signal received from a CO₂ sensor indicates decreased CO₂ level.

12. A lighting controller (110) according to any of claims 1 to 11, further arranged to
obtain one or more images from one or more cameras (136) arranged to capture images of the space;
carry out image analysis to estimate the number of people depicted in the one or more images; and
adjust the relative duration of at least one working period in dependence of the estimated number of people.

13. A lighting controller (110) according to any of claims 1 to 12, further arranged to
receive an indication of a starting time for provision of the light output in accordance with the lighting schedule;
initiate provision of the light output at said starting time in response to a sensor signal received from a presence sensor (134) indicating presence of one or more persons in the space.

14. A lighting controller (110) according to any of claims 1 to 13, further arranged to
receive an indication of a starting time for provision of the light output in accordance with the lighting schedule;
obtain one or more images from one or more cameras (136) arranged to capture images of the space;
carry out image analysis to estimate the number of people depicted in the one or more images; and
initiate provision of the light output at said starting time in response to image analysis indicated presence of one or more persons in the space.

15. A method for controlling light output from one or more luminaires (120) provided for illuminating a space, the method comprising:
obtaining a default lighting schedule that defines respective predefined durations for a sequence of alternating working periods and breaks, where the duration defined for a break in relation to the duration defined for the immediately preceding working period of the sequence increases over time; and
controlling the one or more luminaires (120) to change at least one characteristic of their light output upon the default lighting schedule indicating a transition from a working period to a break and upon the default lighting schedule indicating a transition from a break to a working period.
